# EUROPEAN PATENT APPLICATION

(11) **EP 0 570 016 A1**
(43) Date of publication of application: **18.11.1993**
(21) Application number: 93108013.9
(22) Date of filing: 17.05.1993
(51) Int. Cl.: A61F 13/15

(54) **Shaped absorbent structure, product incorporating same and method of forming same**

(30) Priority: 15.05.1992 US 883637
(71) Applicant: KIMBERLY-CLARK CORPORATION, Neenah, Wisconsin 54956-0349 (US)
(72) Inventor: Schleinz, Alan Francis, Appleton, WI 54914 (US); Rollins, Neal Alan, Menasha, WI 54952 (US); Conger, Gary Lee, Menasha, WI 54952 (US)
(74) Representative: Diehl, Hermann O. Th., Dr.

(57) **Abstract**

A shaped absorbent structure (18) includes a batt (21) of absorbent material with a front region (30) terminating in a first longitudinal end (31), a back region (32) terminating in a second longitudinal end (33), and a crotch region (35). First and second sides (37,39), which each have a curved shape, extend between the first and second longitudinal ends (31,33). Regions (50) of relatively high compression of the batt (21) extend substantially continuously along each of the first and second sides (37,39), inboard of each of the sides, and track the curved shapes of the respective first and second sides. The absorbent structure (18) can be positioned in an outer shell (12) and an acquisition layer (20) as well as a bodyside liner (22) are positioned above same to make up an absorbent structure (10).

## Description

The present invention relates generally to an absorbent structure, a product incorporating same and a method of forming same.

Absorbent structures are utilized in a wide variety of disposable products to absorb and contain urine and other body exudates. For example, disposable products incorporating absorbent structures have proven useful in the fields of infant care, child care, feminine care, and adult incontinence. The commercially available disposable products in these fields are generally unitary, preshaped or prefolded, and comprised of a liquid pervious bodyside liner, a liquid impervious backing sheet, and an absorbent structure disposed between the bodyside liner and the backing sheet. Absorbent structures commonly include natural or synthetic hydrophilic fibers, as well as high-absorbency materials.

Various types of absorbent structures have been devised in an attempt to obtain a proper fit and good leakage protection. Where the absorbent structures include high-absorbency materials, they must be designed to prevent the materials from coming into contact with the user. One example of an absorbent structure containing high-absorbency material is disclosed in U.S. Patent No. 5,043,206 to Ternstrom. The absorbent structure in the Ternstrom patent includes a first layer and a second layer, where high-absorbency material is applied to the second layer. The second layer is compressed to a sufficiently high density in order to fix the high-absorbency material in position within the absorbent structure.

Another absorbent structure, which is intended to contain powdered chemicals, is disclosed in U.S. Patent No. 2,078,300 to Williams. The absorbent pad disclosed in the Williams patent is formed of a multiplicity of layers of absorbent material and incorporates powdered chemicals. The layers of the pad are cut so that the cut edges of the pad are sealed together, in an attempt to retain the chemicals within the pad.

One problem in the art is that the known methods of containing high-absorbency material may have disadvantageous absorbency ramifications. For example, general compression of an absorbent structure to control the movement of high-absorbency material tends to decrease the potential absorbency of the structure. Other methods of containing high-absorbency material include chemically bonding the high-absorbency material to fibers within the absorbent structure, but this may also decrease the potential absorbency of the structure. Furthermore, shearing the edges of an absorbent structure has not proven to be an effective way of containing high-absorbency material.

A related problem in the art is due to the fact that disposable products sometimes include concave, liquid impermeable shells, and, in order to match the shape of the wearer, such shells have been formed with elaborately curved contours. Previously, the absorbent structures for such shaped shells were generally rectangular. This rectangular shape of the absorbent structure necessitated transversely compressing the absorbent structure in order for the absorbent to fit within the shell. U.S. Patents No. 5,030,229 to Yang, and 4,677,810 to Spano, for example, disclose such rectangular absorbent structures and special apparatus for transversely compressing the structure.

What is lacking and needed in the art is an absorbent structure that can be specially-shaped with body-conforming contours, and that effectively contains high-absorbency material without sacrificing the overall absorbent capacity of the structure.

In response to the discussed difficulties and problems encountered in the prior art, a new shaped absorbent structure, a product incorporating same and a method of making same have been developed. Specific aspects of the shaped absorbent structure are evident from independent claim 1, specific aspects of the product are evident from claim 19 and a specific method of making same is evident from independent claim 28. Further advantageous features, aspects and details of the invention are evident from the dependent claims, the description and the drawings. The claims are intended to be understood as a first non-limiting approach of defining the invention in general terms.

The invention provides a shaped absorbent structure for use in an incontinence garment, a disposable diaper, a training pant, a menstrual pad, or the like, and a method of forming a shaped absorbent structure.

A shaped absorbent structure according to a specific aspect of the present invention generally includes a batt of absorbent material. The batt is shaped to form a front region terminating in a first longitudinal end, a back region terminating in a second longitudinal end, and a crotch region therebetween. First and second sides of the batt extend between the longitudinal ends. Each of these first and second sides has a curved shape. The batt also includes regions of relatively high compression which extend substantially continuously along each of the first and second sides. Located inboard of each of the first and second sides, these regions of relatively high compression track the curves of the respective first and second sides. The regions of relatively high compression have a greater density and a reduced thickness compared to portions of the batt that are located inboard of the regions of relatively high compression.

The shaped absorbent structure of the present invention provides numerous advantages, resulting in the efficient utilization of the absorbent material. In the event that the batt of absorbent material is formed as an airlaid mixture of wood pulp fluff and high-absorbency material, the substantially continuous regions of relatively high compression trap the high-absorbency material within the batt. Absorbent structures of the present invention eliminate the need for excessive overall compression, chemical bonds, tissue wraps or other less-advantageous methods which have been used to date to control distribution of high-absorbency material.

In another aspect of the invention, the first and second longitudinal ends and the first and second sides define a rim that extends completely around the batt. The longitudinal ends may also have a curved shape. A single region of relatively high compression extends substantially continuously around the batt inboard of the rim, tracking the curves of the ends and the sides.

This aspect results in a shaped absorbent structure that may be easily incorporated into an absorbent product, such as a feminine guard or pad. The region of relatively high compression may extend substantially continuously around the rim of the batt. The resulting structure is thereby given considerable rigidity in both the longitudinal and transverse directions. Manipulation of the absorbent structure, such as for insertion into a polymeric foam shell, is greatly simplified. Furthermore, products incorporating the shaped absorbent structure may experience reduced amounts of leakage, because the region of relatively high compression tends to space the periphery of the absorbent structure away from the body-contacting materials of the product, particularly the liner.

In particular embodiments of the invention as set forth herein, the region of relatively high compression is located from about 0 to about 50 millimeters from the rim. Further, the region of relatively high compression has a density of from about 0.05 to about 2.0 grams per cubic centimeter, whereas the portions of the batt located further inboard have a density of from about 0.01 to about 1.0 grams per cubic centimeter. Even though the density ranges for the region of relatively high compression and the inboard areas overlap, it should be understood that for any given absorbent structure of the present invention, the region of relatively high compression will have a greater density than areas located inboard of the region. The thickness of the region of relatively high compression may be from about 0.1 to about 10 millimeters, while the portions of the batt located further inboard have a thickness of from about 1.0 to about 100 millimeters. The invention as described results in a shaped absorbent structure with desirable fluid management characteristics. The region of relatively high compression is thought to inhibit the movement of fluids to the ends and sides of the batt. Fluids reaching the region of relatively high compression instead tend to be drawn along the substantially continuous region, only to be desorbed by other portions of the batt.

The present invention also provides a method of forming a shaped absorbent structure. According to a specific aspect the method includes the steps of providing a web of absorbent material and selecting a desired shaped structure to be separated from the web. The desired shaped structure has a front region terminating in a first longitudinal end, a back region terminating in a second longitudinal end, and a crotch region between the front and back regions. It also has first and second sides extending between the longitudinal ends. The first and second longitudinal ends and the first and second sides have curved shapes. The first and second longitudinal ends and the first and second sides define a rim extending completely around and defining the desired shaped structure. The method also includes the step of forming a region of relatively high compression in the web. The region of relatively high compression extends substantially continuously around the rim, inboard of the rim. The region of relatively high compression tracks the curved shapes of the first and second longitudinal ends and the first and second sides. The region has a greater density and a reduced thickness compared to portions of the web located inboard of the region of relatively high compression. The method also includes cutting the web along the rim to separate the desired shaped structure from the web. This method provides a convenient means of producing a shaped absorbent structure having the aforementioned advantages.

As can be seen from the foregoing comments, it is an aspect of the present invention to provide a shaped absorbent structure that efficiently absorbs fluidic waste that may be discharged by a wearer.

It is another aspect of the invention to provide a shaped absorbent structure that incorporates high-absorbency material, without having to excessively overall compress, chemical bond, or tissue wrap the structure in order to contain the high-absorbency material.

It is still another aspect of the invention to provide a shaped absorbent structure that may be easily manufactured and assembled into an absorbent product.

It is yet another aspect of the invention to provide a shaped absorbent structure that reduces the potential for fluid migration to the rim of the structure.

The foregoing and other aspects, features and advantages of the present invention will appear from the following description. In the description, reference is made to the accompanying drawings which illustrate preferred embodiments of the invention.

Fig. 1 is a perspective view of an absorbent product according to the present invention.

Fig. 2 is an exploded perspective view of the absorbent product of Fig. 1, showing a shaped absorbent structure and other components of the product.

Fig. 3 is an enlarged view in section taken generally from the plane of the line 3-3 of Fig. 1.

Fig. 4 is a schematic perspective view illustrating operation of equipment used to produce shaped absorbent structures of the present invention.

Fig. 5 is an enlarged view in section similar to Fig. 3, but showing an alternate embodiment of an absorbent product according to the present invention.

Fig. 6 is an exploded perspective view of a shaped absorbent structure for use in the absorbent product as shown in Fig. 5.

Fig. 7 is a view in longitudinal section of the shaped absorbent structure of Fig. 6.

Fig. 8 is an enlarged view in section similar to Figs. 3 and 5, but showing another alternate embodiment of a shaped absorbent structure in an absorbent product according to the present invention.

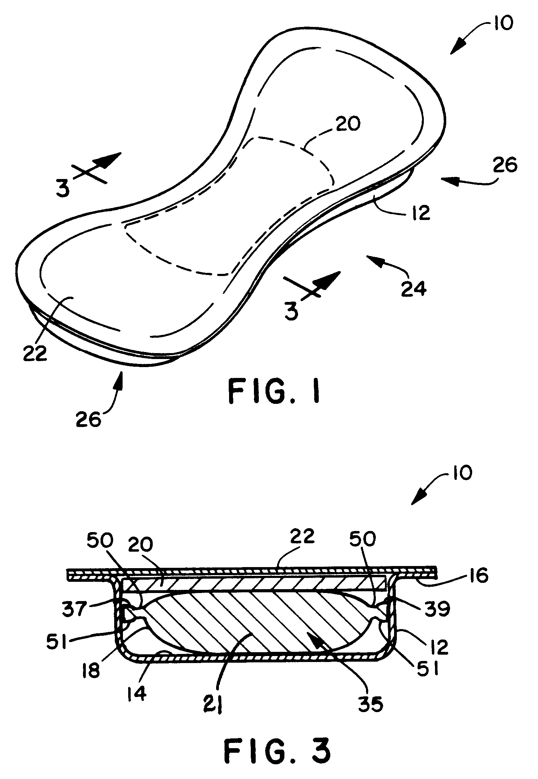

With reference to Figs. 1-3, a disposable absorbent product 10 according to the present invention is shown for purposes of illustration as a feminine incontinence product. The invention may also be incorporated in other types of disposable absorbent products, such as diapers, training pants or menstrual products.

The product 10 as shown includes four separate components. Specifically, the product 10 includes an outer shell 12, which defines a basin 14 and a rim 16. The basin 14 has a length, width, and volume. Contained within the basin 14 is a shaped absorbent structure 18. Superposed on top of the absorbent structure 18 is an acquisition layer 20. The acquisition layer 20 is also located within the basin 14 and sits on top of and in fluid communication with the shaped absorbent structure 18. In the illustrated embodiment, dots of adhesive 19 secure the acquisition layer 20 to the shaped absorbent structure 18. Finally, a body side liner 22 covers the basin 14 and is attached to the outer shell 12 along the periphery of the rim 16. Thus, the acquisition layer 20 is located between the liner 22 and shaped absorbent structure 18.

The components discussed above will now be described in more detail, with various preferred embodiments of the present invention being discussed. The outer shell 12 is formed from a flexible material that is liquid-impervious. Exemplary of materials suitable for use in forming the outer shell 12 are various thermoplastic or thermosetting polymeric resins such as polyethylene, polypropylene, polyurethane, polyesters, and the like. In one preferred embodiment of the present invention, the outer shell 12 is formed from a thin layer of polyethylene foam commercially available from Voltek Inc. of Lawrence, Massachusetts, under the trade designation Volara. Other thermoplastic or thermosetting polymeric foams are suitable for use in the present invention.

In one preferred embodiment, the outer shell 12 possesses sufficient structural rigidity to form a stand-alone, three-dimensional shell. In such a case, the outer shell suitably has a thickness of from ca. 0.25 - 6.35 mm (about 0.01 inch to about 0.25 inch) , and preferably from ca. 0.76 - 3.18 mm (about 0.03 inch to about 0.125 inch). It is also believed that the outer shell 12 could be formed from a material lacking sufficient structural rigidity to form a stand-alone, three-dimensional shell. Exemplary of such a material would be a thin, substantially liquid impermeable web or sheet of plastic film such as polyethylene, polypropylene, polyvinyl chloride or similar material having a thickness of ca. 0.03 mm (about 0.001 inch). Alternately, the outer shell 12 may comprise a nonwoven, fibrous web which has been suitably constructed and arranged to be substantially liquid impermeable. In any event, the outer shell 12 is generally sufficiently flexible to readily conform to pressures exerted on it during use by a wearer. The outer shell 12 can be formed from a variety of manufacturing processes such as thermoforming, vacuum forming, injection molding, mechanical forming, and the like.

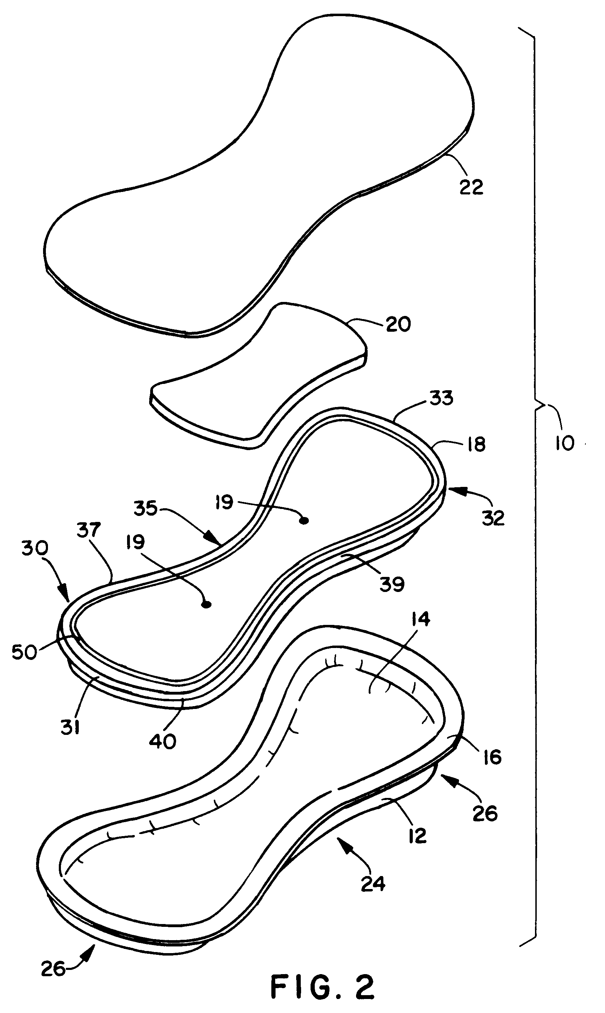

For improved comfort and effective operation, the outer shell 12 desirably has a relatively-narrow section 24 between longitudinal end sections 26 (Figs. 1 and 2). In the illustrated embodiment, the outer shell 12 has a centrally-located section 24 which is narrower than the longitudinal end sections 26. While the difference in width between the central section 24 and the longitudinal end sections 26 can vary, it is desirable for a central section to have a width which is from about 10 to about 70 percent, and preferably from about 25 to about 50 percent less than the width of at least one of the longitudinal end sections 26 of the product 10.

For a desirable fit as a feminine incontinence product, the outer shell 12 suitably has a length of from ca. 10.16 - 30.48 cm (about 4 to about 12 inches), a width of from ca. 3.30 - 17.78 cm (about 1.3 to about 7 inches), and a depth of from ca. 0.64 - 6.35 cm (about 0.25 to about 2.5 inches). In the illustrated embodiment, the product 10 possesses the same width at each longitudinal end section 26. Moreover, the narrowest portion of the product 10 is located generally in its center. Nonetheless, it is understood that the longitudinal end sections 26 of the product 10 may have the same or different widths, and that the narrowest portion of the product 10 may be offset towards either end section. In one preferred embodiment, the product 10 has a length of ca. 24.13 cm (about 9.5 inches), a width at the longitudinal end sections 26 of ca. 10.80 cm (about 4.25 inches), and a width in the central section 24 of ca. 7.32 cm (about 2.88 inches).

The outer shell 12 desirably defines a basin 14 and a rim 16. As can be seen from Figs. 1 and 2, the length of the illustrated product 10 comprises the length of the basin 14 plus the width of the rim 16 at both longitudinal end sections 26 of the outer shell 12. The width of the illustrated product 10 along any transverse line comprises the width of the basin 14 plus the width of the rim 16 on each longitudinal side of the outer shell 12. The width of the rim 16 is suitably from ca. 1.52 - 38.10 mm (about 0.06 to about 1.5 inches), and preferably from ca. 6.35 - 19.05 mm (about 0.25 to about 0.75 inches). It is generally preferred that the rim 16 be continuous around the periphery of the basin 14 and have a generally uniform width. The depth of the product 10 comprises the depth of the basin plus the thickness of the outer shell 12.

The shaped absorbent structure 18 is generally fibrous and adapted to absorb and hold body fluids such as urine and menses. The absorbent structure 18, which is located within the basin 14, fills from about 10 to about 95 volume percent, and preferably from about 50 to about 85 volume percent, of the volume of the basin.

The absorbent structure 18 suitably has a thickness of from ca. 3.18 - 38.10 mm (about 0.125 inch to about 1.5 inches), preferably from ca. 6.35 - 25.40 mm (about 0.25 inch to about 1.0 inch). The absorbent structure 18 suitably comprises a batt or pad of airlaid cellulosic fibers commonly referred to as wood pulp fluff. Suitable types of wood pulp fluff are available from Kimberly-Clark Corporation of Neenah, Wisconsin. Conventional pads of wood pulp fluff can have a density ranging from about 0.05-0.20 grams per cubic centimeter and are sufficiently flexible to readily conform to pressures exerted against the absorbent structure 18 during use. The absorbent structure 18 may also comprise a pad of coform material composed of a mixture of cellulosic fibers and synthetic polymer fibers. For example, the coform material may comprise an airlaid blend of cellulosic fibers and meltblown polyolefin fibers such as polyethylene or polypropylene fibers. Additionally, it is possible for the absorbent structure 18 to be formed completely from meltblown polymeric fibers. The absorbent structure 18 can optionally include substantially hydrophilic tissue or other nonwoven sheets (not shown) on its major surfaces to help maintain the integrity of the fibrous structure. Such tissue sheets typically comprise an absorbent cellulosic material, such as creped wadding or high wet-strength tissue.

The absorbent structure 18 may also include an effective amount of an inorganic or organic high-absorbency material (also known as superabsorbent material) to enhance the absorptive capacity of the absorbent structure. For example, the absorbent structure can include from about 0 to about 95 weight percent of high-absorbency material and preferably includes from about 10 to about 30 weight percent of high-absorbency material to provide more efficient performance.

Suitable inorganic, high-absorbency materials include, for example, absorbent clays and silica gels. Organic high-absorbency materials can include natural materials such as agar, pectin, guar gum, and peat moss, as well as synthetic materials such as synthetic hydrogel polymers. Such hydrogel polymers include, for example, carboxymethylcellulose, alkali metal salts of polyacrylic acids, polyacrylamides, polyvinyl alcohol, ethylene maleic anhydride copolymers, polyvinyl ethers, hydroxypropylcellulose, polyvinyl morpholinone, polymers and copolymers of vinyl sulfonic acid, polyacrylates, polyacrylamides, polyvinyl pyridine, and the like. Other suitable polymers include hydrolyzed acrylonitrile grafted starch, acrylic acid grafted starch, and isobutylene maleic anhydride copolymers and mixtures thereof. The hydrogel polymers are preferably lightly crosslinked to render the material substantially water insoluble. Crosslinking may, for example, be by irradiation or by covalent, ionic, Van der Waals, or hydrogen bonding. Suitable materials are available from various commercial vendors, such as Dow Chemical Company, Celanese Corporation, Allied-Colloid Inc., and Stockhausen Inc. Typically the high-absorbency material is capable of absorbing at least about 15 times its weight in water, and preferably is capable of absorbing at least about 25-50 times its weight in water.

The high-absorbency material can be distributed or otherwise incorporated into the absorbent structure 18 employing various techniques. For example, the high-absorbency material can be substantially uniformly distributed in the mass of fibers comprising the absorbent structure. The high-absorbency material can also be non-uniformly distributed among the fibers to form, for example, a layer or a generally continuous gradient with either an increasing or decreasing concentration of superabsorbent material. In one preferred embodiment, the high-absorbency material forms an increasing gradient from that portion of the absorbent structure closest the body side liner 22 to that portion farthest from the body side liner.

As best seen in Fig. 2, the shaped absorbent structure 18 is formed with a front region 30 and a back region 32. The front and back regions 30 and 32 terminate, respectively, in first and second longitudinal ends 31 and 33. The front and back regions 30 and 32 are longitudinally separated by a crotch region 35. The shape of the crotch region 35 is determined principally by first and second sides 37 and 39 of the structure 18 which extend between the first and second longitudinal ends 31 and 33. A rim 40 represents the edge of the absorbent structure 18 and extends completely around the structure which contains an absorbent batt 21. The rim 40 is defined by the first and second longitudinal ends 31 and 33 and the first and second sides 37 and 39.

The absorbent structure 18 is most desirably formed with a shape that matches the shape of the basin 14 of the outer shell 12. In this situation, the absorbent structure 18 may be easily placed within the outer shell 12, and the structure fills the full length and width of the basin 14.

In particular, the first and second sides 37 and 39 of the absorbent structure 18 are formed with a curved shape. This curved shape enables the crotch region 35 to be more narrow than one or both of the front and back regions 30 and 32. By curved-shaped sides 37 and 39, it is meant that the sides include at least one arc portion of an imaginary circle, the center of which is not located on the sides. For added comfort, it is desirable for the sides 37 and 39 to be formed of a shape including a plurality of such arc portions. In this case, the arc portions in the crotch region 35 form a concave surface, and the arc portions adjacent at least one of the ends 31 or 33 form a convex surface (see Fig. 2).

In the embodiment illustrated, the first and second longitudinal ends 31 and 33 are also formed with a curved shape. By curved-shaped ends 31 and 33, it is meant that the ends include at least one arc portion of an imaginary circle, the center of which is not located on the ends. Preferably, and as illustrated, the ends 31 and 33 are formed with a convex shape, such that the ends curve or bulge outward. In this way, the shape of the front and back regions 30 and 32 of the absorbent structure 18 is similar to the shape of the longitudinal end sections 26 of the outer shell 12.

As previously noted, the longitudinal end sections 26 of the outer shell 12 may have different widths, and the narrowest portion of the shell may be offset towards one end section. In such a case, the curved shapes of the first and second sides 37 and 39 and the first and second longitudinal ends 31 and 33 may be selected to correspond to the shape of the basin 14 of the shell 12.

The shaped absorbent structure 18 is also formed with a region of relatively high compression, which is represented in the drawings by reference numeral 50. The region of relatively high compression 50 is an area of the absorbent structure 18 which has been compressed to have a greater density and a reduced thickness compared to portions of the absorbent structure located inboard of the region of relatively high compression. The inboard portions are those areas of the absorbent structure 18 that are located more toward the center of the structure than the region of relatively high compression 50. The region 50 suitably has a density of from about 0.05 to about 2.0 grams per cubic centimeter, and preferably from about 0.2 to about 1.5 grams per cubic centimeter. The region 50 suitably has a thickness of from about 0.1 to about 10.0 millimeters, and preferably of from about 0.25 to about 5.0 millimeters.

By comparison, the portions of the absorbent structure 18 located inboard of the region of relatively high compression 50 have a density of from about 0.01 to about 1.0 grams per cubic centimeter, and preferably of from about 0.01 to about 0.5 grams per cubic centimeter. Also, the portions of the absorbent structure 18 located inboard of the regions of relatively high compression 50 have a thickness of from about 1.0 to about 100 millimeters, and preferably of from about 1.0 to about 50 millimeters.

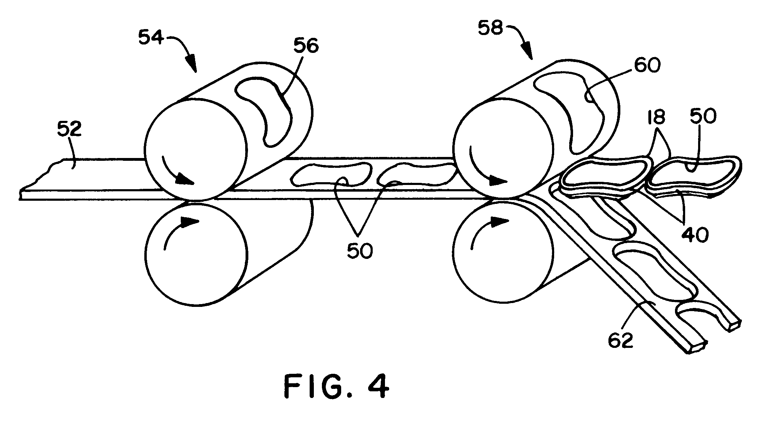

The region of relatively high compression 50 is located inboard of the rim 40, and is preferably spaced from the rim. As illustrated in Fig. 3, this causes the portions (designated reference numeral 51) of the absorbent structure 18 located outboard of the region 50 to have a greater thickness and a lower density compared to the region 50 itself. It has been found that the region of relatively high compression suitably has a width of from about 0.5 to about 25 millimeters, and preferably of from about 1.0 to about 10.0 millimeters. Further, the outboard limit of the region of relatively high compression is suitably spaced from the rim 40 by from 0 to about 50 millimeters, and preferably by from about 0.5 to about 10.0 millimeters.

By way of illustration, an absorbent structure 18 could be formed such that the region of relatively high compression 50 has a density of about 0.9 grams per cubic centimeter and a thickness of about 1 millimeter. Also, the portions of the absorbent structure 18 located inboard thereof have a density of about 0.05 grams per cubic centimeter and a thickness of about 10 millimeters. The region of relatively high compression 50 could be approximately 2.5 millimeters wide, and spaced inward from the rim 40 by about 3.2 millimeters.

The incorporation of the region of relatively high compression 50 in the absorbent structure 18 provides several advantages. For instance, the region 50 increases the structural integrity of the shaped absorbent structure 18 in both its longitudinal and transverse dimensions. This increase in structural integrity makes the absorbent structure 18 easier to handle during assembly of the product 10.

Another advantage is that the region of relatively high compression 50 reduces the loss of high-absorbency material from the shaped structure 18. As described hereinafter, the curved sides 37 and 39 and the curved ends 31 and 33 of the absorbent structure 18 are cut from a web of absorbent material. The cut edges of the structure would otherwise experience a high degree of loss of high-absorbency material. The region of relatively high compression 50, however, provides a barrier to resist movement and escape of such high-absorbency material.

Additionally, the region of relatively high compression 50 promotes the movement of fluids along the rim 40, rather than allowing the escape of fluids from the absorbent structure 18 at the rim. Liquids within the absorbent structure 18 that move toward the region 50 tend to be drawn by capillary pressure of the densified region 50 along the rim 40. When the region 50 is spaced inboard from the rim 40 (see Figs. 2 and 3), the liquid tends not to move toward the rim, which is in a thicker, less-heavily compressed portion 51 of the absorbent structure 18.

With reference to Fig. 2, the region of relatively high compression 50 desirably extends continuously around the rim, tracking the shapes of the first and second ends 31 and 33 and the first and second sides 37 and 39. Optionally, the region may actually comprise two regions of relatively high compression (not shown), which extend continuously along each of the first and second sides 37 and 39 and track the curved shapes of the sides. In this case, the regions (not shown) provide longitudinal integrity or stiffness to the absorbent structure 18, reduce the loss of high-absorbency material from the sides, and promote fluid movement along the sides 37 and 39.

Also optionally, the region (or regions) of relatively high compression 50 may extend substantially continuously rather than continuously along the rim 40. By substantially continuously, it is meant that a series of regions of relatively high compression may be positioned closely adjacent one another along the rim 40 to track the shape of the sides and ends. Provided such a series of regions of relatively high compression are spaced sufficiently close together, an absorbent structure may also provide the previously-mentioned benefits.

Also present within the basin 14 is the acquisition layer 20. The acquisition layer 20 can be or can contain any suitable material for managing, accommodating, permitting, or directing rapid and/or sudden flow of urine and/or other excreted or discharged liquid therethrough and into contact with the shaped absorbent structure 18. For example, the acquisition layer 20 may comprise a substantially hydrophobic transport material such as nonwoven polypropylene, polyethylene, polyester, blends thereof, and the like. Such substantially hydrophobic transport materials preferably contain or are treated with a suitable surfactant to increase their initial wettability and thus increase the rate of movement of liquid into the absorbent structure 18 during initial insult or discharge of liquid into the product 10.

More preferably, however, the acquisition layer 20 is structurally adapted to be able to rapidly absorb multiple insults of a relatively small amount of urine delivered at a relatively low velocity, multiple insults of a relatively large amount of urine delivered at a relatively high velocity, and menses. In particular, the acquisition layer 20 preferably comprises a fibrous web of dry resilient fibers with at least about 30 weight percent of the fibers being hydrophilic. As used herein, a fiber will be considered to be hydrophilic when the fiber possesses a water-in-air contact angle of less than 90°, and preferably of less than about 45°.

Those skilled in the art will recognize suitable hydrophilic fibers for forming the acquisition layer 20 of the present invention. Exemplary of suitable fibers are inherently hydrophilic fibers such as rayon, cotton, and the like, as well as inherently hydrophobic fibers such as polyolefins and polyesters which have been rendered hydrophilic by surface treatment with a nonfugitive surface active agent. An inherently hydrophobic fiber will be considered to be hydrophilic when the fiber exhibits a water-in-air contact angle below 90°, preferably below about 45°, for three successive measurements with drying between each measurement.

When less than about 50 weight percent of the fibers of the acquisition layer 20 are hydrophilic, it is desirable that the hydrophilic fibers be localized in discrete areas such that the discrete areas comprise at least 50 weight percent hydrophilic fibers based an total weight of such discrete areas. For example, clusters of hydrophilic fibers can be formed into a web of hydrophobic meltblown fibers such that the hydrophilic fibers form discrete areas comprising a majority of hydrophilic fibers.

The acquisition layer 20 temporarily holds the absorbed liquid and then releases the liquid to the absorbent structure 18. That is, the absorbent structure 18 is generally capable of desorbing the acquisition layer 20 to some degree. As a general rule, the absorbent structure 18 is not able to absorb multiple insults of a liquid as rapidly as the acquisition layer 20 and the acquisition layer is not able to hold as much liquid as the absorbent structure. By having both the absorbent structure 18 and the acquisition layer 20 present in the product 10, as is presently preferred, the product is able to both rapidly absorb multiple insults of a liquid and to hold relatively large quantities of the liquid.

The acquisition layer 20 is preferably, although not necessarily, formed from fibers having a compression recovery value when dry of at least about 60 percent, and preferably of at least about 80 percent. The compression recovery value is a measure of the resilience of the material and is determined by measuring the original thickness of the acquisition layer 20 under a restraining pressure of 0.47 kPa (0.068 pounds per square inch). The acquisition layer 20 is then subjected to a compression force of 3.45 kPa (0.5 pounds per square inch) for 60 seconds. This compression force is then removed and, after four minutes, the recovery thickness of the material is measured under the original pressure of 0.47 kPa (0.068 psi). The compression recovery value is then determined by dividing the recovery thickness by the original thickness and is reported as percent recovery.

The acquisition layer 20 suitably has a thickness of from ca. 2.54 - 25.40 mm (about 0.1 inch to about 1.0 inch), and preferably of from ca. 2.54 - 12.70 mm (about 0.1 inch to about 0.5 inch), and most preferably of from ca. 3.81 - 7.62 mm (about 0.15 inch to about 0.3 inch). The acquisition layer 20 suitably has a basis weight of from about 40 to about 250 grams per square meter, and preferably of from about 80 to about 130 grams per square meter. Additionally, the acquisition layer 20 has a pore size which allows the acquisition layer to rapidly receive a liquid applied thereto in large quantities at a relatively high velocity and release the liquid to the absorbent structure 18.

The acquisition layer 20 is present within the basin 14 and sits atop the absorbent structure 18. The acquisition layer 20 suitably fills from about 5 to about 50 volume percent, and preferably from about 5 to about 30 volume percent, of the basin 14. In one preferred embodiment, the acquisition layer 20 is adhesively attached to the absorbent structure 18 by dots or lines of hot melt, pressure sensitive, or other types of adhesives.

In one preferred embodiment of the present invention, the acquisition layer 20 is formed from rayon fibers having an average fiber fineness of from about 0.22 tex to about 1.66 tex (about 2 denier to about 15 denier), and preferably of from about 0.61 tex to about 0.89 tex (about 5.5 denier to about 8.0 denier). The web is formed by airlaying the fibers and then needling the air laid web. The web has a latex binder comprising vinyl acetate applied thereto to adhere certain fiber-fiber intersections together. Exemplary of such a material is a rayon web, commercially available from Sackner Products Inc., Grand Rapids, Michigan, under the trade designation SN-92. An acquisition layer 20 of this type desirably has a thickness of ca. 6.35 mm (about 0.25 inch) and a basis weight of about 120 grams per square meter.

The acquisition layer 20 can extend along the entire length and width of the basin 14. Preferably, however, the acquisition layer 20 has a length which is less than the length of the basin 14 and a width which is substantially the same as the width of the basin, in the crotch region 35. Most preferably, the acquisition layer 20 is located only in the central 1/3 to 2/3 of the length of the basin 14. Applicants have discovered that when the acquisition layer 20 is located only in the central 1/3 to 2/3 of the length of the basin 14, liquid applied to the acquisition layer can transfer through the length of the acquisition layer, out the ends of the acquisition layer and be absorbed by the absorbent structure 18 at portions of the absorbent structure remote from that area most likely to receive additional insults of a liquid. By having the length of the acquisition layer 20 be 1/3 to 2/3 of the length of the basin 14, the likelihood of leakage due to liquid transfer to the end edges of the basin is lessened.

The acquisition layer 20 is in fluid communication with the absorbent structure 18 such that a liquid present in the acquisition layer is drawn from the acquisition layer into the absorbent structure. To accomplish this, there must be a capillary pressure gradient between the acquisition layer 20 and the absorbent structure 18, such that a liquid present in the acquisition layer is drawn from the pores of the acquisition layer into the pores of the absorbent structure. Those skilled in the art will recognize that a number of variables influence the capillary pressure including the surface tension of a liquid, the liquid-fiber contact angle and the capillary radius. Those skilled in the art can adjust the liquid-fiber contact angle and capillary radius in order to ensure the required degree of transfer between the acquisition layer 20 and the absorbent structure 18. In this manner, the absorbent structure 18 can desorb the acquisition layer 20 to prepare the acquisition layer to receive subsequent insults of a liquid and to provide the surface of the product 10 in contact with the user with a relatively dry feel.

A body side liner 22 covers the top of the basin 14 and is attached to the outer shell 12 along the rim 16. The body side liner 22 is typically composed of a liquid-permeable, substantially hydrophobic, fibrous material such as spunbonded web composed of synthetic polymer filaments. Alternatively, the body side liner 22 may comprise a meltblown web or a bonded-carded web composed of synthetic polymer filaments. Suitable synthetic polymers include, for example, polyethylene, polypropylene, polyesters, and combinations thereof. The body side liner 22 suitably has a basis weight of from about 10 to about 30 grams per square meter, and preferably of from about 12 to about 20 grams per square meter. An apertured film may also be employed as the body side liner.

Applicants have found that it is preferable to have the body side liner 22 formed from a material that is hydrophobic and that possesses a passage volume of 40 volume percent, and more preferably of 60 percent. As used herein, passage volume refers to the volume percent of a drop of distilled water which passes through the liner when applied at a velocity of 198.12 cm (6.5 feet) per second. The method by which passage volume is determined is set forth in detail in copending application 07/598,272 filed October 16, 1990 (EP 0 483 592), titled Disposable Feminine Guard and incorporated herein by reference to the extent that it is consistent herewith. Applicants have found that body side liners having a passage volume of at least 40 volume percent are able to pass urine applied in relatively small amounts at low velocity and menses into the acquisition layer 20. Body side liners not able to meet this standard tend to cause urine applied in small amounts at a low velocity and menses to pool on their surface and run off. Body side liners may be treated with a surface active agent to increase their passage volume.

In one preferred embodiment, the body side liner 22 is a bonded carded web containing 50 weight percent 0.33 tex (3 denier) polypropylene fibers and 50 weight percent of a polyethylene sheath polypropylene core fiber (50 weight percent polyethylene, 50 weight percent polypropylene). The polypropylene fibers are formed into a carded web having a basis weight of 8 grams per square meter. The sheath/core fibers are also formed into an 8 gram per square meter carded web. The two carded webs are placed one on top of another and through air bonded. Such a body side liner is commercially available from Chori America Inc., Los Angeles, California, under the trade designation NBF(H).

The body side liner 22 can be attached to the product 10 at rim 16 in a variety of manners known to those skilled in the art. For example, the body side liner 22 may be adhesively bonded to the rim or may be thermally or ultrasonically bonded.

In use, the product 10 is positioned between a wearer's legs in a manner similar to a feminine napkin. The product 10 may be maintained in position due to pressure exerted by the wearer's underclothes or may be attached to the wearer's underclothes with any of a variety of attachment means known to those skilled in the art. For example, the product 10 may be attached to the underclothes of a wearer through the use of an adhesive strip applied to the outer surface of the outer shell. Optionally, other types of attachment systems, such as tapes or hook-and-loop fasteners (not shown), may be employed.

A disposable absorbent product 10 according to the present invention may be constructed by separately forming each of the four elements and uniting them in the following manner. The shaped absorbent structure 18 can be placed into the basin 14 of the outer shell 12. The shape of the absorbent structure 18, as defined by the rim 40 is preferably sized to fit within and against the upwardly-extending walls of the basin 14. The acquisition layer 20 may be cut with a width to match the width of the shaped absorbent structure 18 in the crotch region 35. As previously noted, the acquisition layer 20 may be secured to the absorbent structure 18 with dots of adhesive 19 or other suitable means. Thus, the shaped absorbent structure 18 and the acquisition layer 20 are both positioned within the basin 14. The body side liner 22 may be cut with a shape that matches the shape of the rim 16 of the outer shell 12. Again as previously noted, the periphery of the body side liner 22 may be attached to the rim 16 using adhesives, thermal bonds, ultrasonic bonds or other suitable means.

A preferred method of forming the shaped absorbent structure 18 is illustrated schematically in Fig. 4. Initially, a web 52 of absorbent material, which may include an air-laid mixture of wood pulp fluff and high-absorbency material, is inserted between rotating compression and anvil rolls 54. The compression and anvil rolls rotate in the direction indicated by the arrows in Fig. 4, and the compression roll includes a series of raised patterns 56. As the web 52 of absorbent material passes between the compression and anvil rolls 54, the raised patterns 56 on the compression roll more heavily compress selected area(s) of the web 52 to form the region(s) of relatively high compression 50. By way of illustration, the primary surfaces of the compression and anvil rolls 54 may be spaced apart by a distance of approximately 50 millimeters. The raised patterns 56 on the compression roll may extend radially outward from its primary surface by approximately 49.9 millimeters, so that the raised patterns 56 form a gap of only about 0.1 millimeter with the anvil roll.

The web 52 of absorbent material is then inserted between cutting and anvil rolls 58, which rotate in the direction indicated by the arrows in Fig. 4. The cutting roll includes a series of raised cutting surfaces 60 which separate the shaped absorbent structures 18 from the residual portion 62 of the web 52. The cutting and anvil rolls 58 are appropriately spaced and timed in relation to the compression and anvil rolls 54 so that the cut rim 40 of each shaped absorbent structure 18 is formed outboard of the region of relatively high compression 50, as previously indicated. The primary surfaces of the cutting and anvil rolls 58 may be spaced apart by approximately 50.0 millimeters, and the raised cutting surfaces 60 on the cutting roll may extend radially outward from the primary surface of the cutting roll by approximately 50.0 millimeters.

The disposable absorbent product 10 incorporating a shaped absorbent structure 18 has other benefits besides those previously mentioned. The region of relatively high compression 50 is generally thinner than portions of the absorbent structure 18 located inboard of the region of relatively high compression. As shown best in Fig. 3, these inboard portions of the absorbent structure 18 tend to be positioned in contact with the acquisition layer 20, which is in turn in contact with the body side liner 22. Conversely, the region of relatively high compression 50 tends to be spaced apart from the acquisition layer 20, and the liner 22. In fact, where the region of relatively high compression 50 extends substantially continuously around the rim 40 of the absorbent structure, the entire periphery of the absorbent structure tends to be spaced apart from the acquisition layer 20, and the liner 22. It is believed that this feature will tend to improve leakage performance of the product 10. Fluid migrating outward within the acquisition layer 20 will be drawn into the absorbent structure 18 and away from the acquisition layer, rather than into the peripheral portions of the acquisition layer that are spaced apart from the region of relatively high compression layer 50. Thus, fluid is drawn into the absorbent structure 18 instead of travelling toward the rim 16 of the product 10, where leakage may occur.

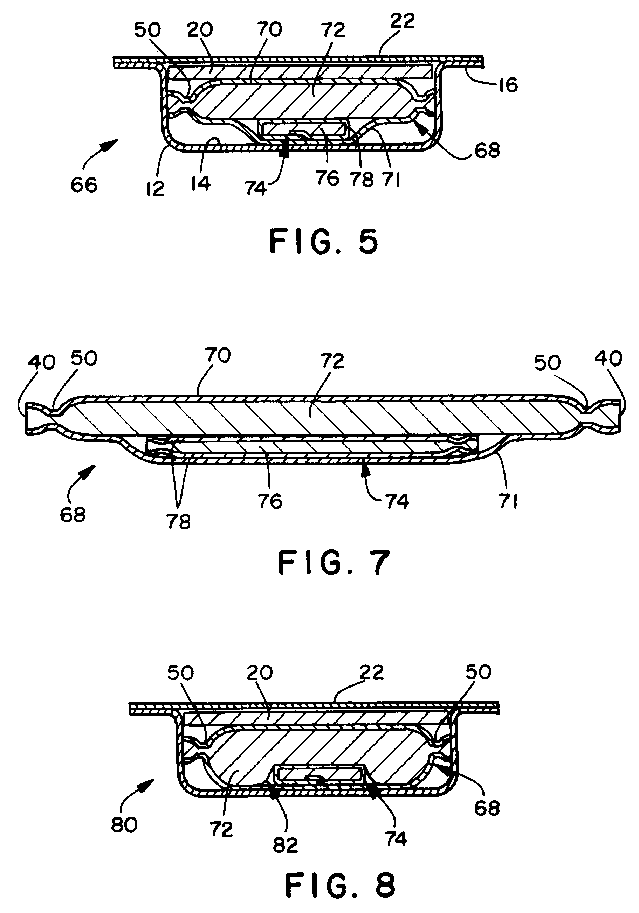

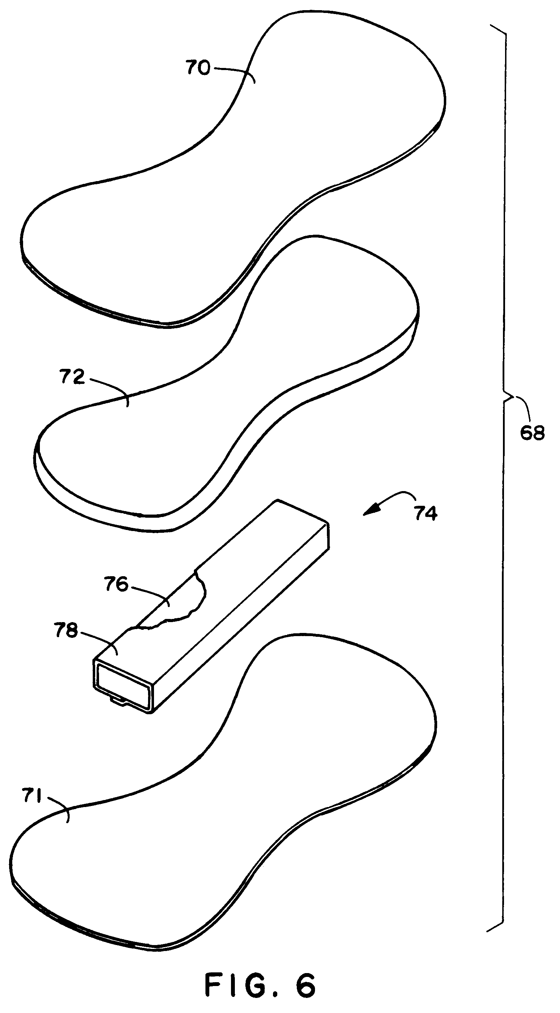

A second embodiment of the present invention is illustrated by the disposable absorbent product 66 in Fig. 5. Components similar to those previously described are labeled with the same reference numeral. A shaped absorbent structure 68 and an acquisition layer 20 are positioned within a basin 14 of a liquid impermeable outer shell 12. A body side liner 22 is attached to a rim 16 of the outer shell 12 to maintain the absorbent structure 68 and the acquisition layer 20 within the basin 14.

The shaped absorbent structure 68 is shown in an exploded perspective view in Fig. 6 and in a longitudinal sectional view in Fig. 7. The shaped absorbent structure 68 includes upper and lower tissue layers 70 and 71, a web or batt of absorbent material 72, and a pledget 74. The pledget 74 includes a web or batt of absorbent material 76 that is relatively smaller in size than the web 72. The pledget 74 may include a tissue or other nonwoven wrap sheet 78 surrounding the relatively small batt of absorbent material 76. Both the larger web 72 and the smaller web 76 may be formed of absorbent materials as previously disclosed, such as an air-laid mixture of wood pulp fluff and high-absorbency materials.

The shaped absorbent structure 68 is constructed in a manner similar to that described in relation to Fig. 4. The relatively small web of absorbent material 76 is initially formed and enveloped within the wrap sheet 78. The wrap sheet 78 may be sealed at its sides and ends around the relatively small web 76 in order to prevent the loss of high-absorbency materials from the web 76. Optionally, the wrap sheet 78 may be sealed only along its longitudinal sides, and the longitudinal ends of both the small web 76 and the wrap sheet 78 may be transversely embossed to frictionally bond the small web 76 and the wrap sheet 78 and to reduce the escape of high-absorbency materials from the longitudinal ends of the small web 76.

The pledget 74 is preferably rectangular in shape and shorter and narrower than the larger web 72. The pledget 74 is centrally positioned against one surface of the larger web 72. The upper tissue layer 70 is positioned against one surface of the larger web 72, and the lower tissue layer 71 is positioned against an opposite surface of the larger web 72 so that the pledget 74 is sandwiched between the larger web 72 and the lower tissue layer 71. The composite structure of the upper and lower tissue layers 70 and 71, the larger web 72 and the pledget 74 are processed through compression and anvil rolls 54 to form a region of relatively high compression 50 (see Figs. 5 and 7). Preferably although not necessarily, the pledget 74 is sized to fit inside the raised patterns 56 in the compression roll. The region of relatively high compression 50 preferably extends substantially continuously around the rim 40 of the absorbent structure 68, inboard of the rim and tracking the shape of the rim. The region 50 has a relatively greater density and a reduced thickness compared to portions of the larger web of absorbent material 72 that are located inboard of the region of relatively high compression 50.

A third embodiment of the present invention is illustrated by the disposable absorbent product 80 in Fig. 8. This disposable absorbent product 80 differs from the product 66 shown in Fig. 5, in that the larger web or batt of absorbent material 72 is formed on one surface with a pocket 82. The pocket 82 represents an area of the larger web 72 with a reduced thickness. Preferably, the pocket 82 is centrally located within the larger web 72 and sized such that the pledget 74 may generally reside within the pocket. The product 80 of Fig. 8 is advantageous in that a relatively high percentage of the volume of the basin 14 can be filled with absorbent material. Particularly, the shaped absorbent structure 68 may fill from about 10 to about 100 volume percent of the volume of the basin 14, and particularly from about 70 to about 80 volume percent of the volume of the basin.

The foregoing detailed description has been for the purpose of illustration. Thus, a number of modifications and changes may be made without departing from the spirit and scope of the present invention. For example, the composition of the shaped absorbent structures may vary greatly. Likewise, the shape, size and components of the shaped absorbent structure may also vary, and as previously noted, the shaped absorbent structures may be incorporated in a wide variety of disposable absorbent products.

## Claims

1. A shaped absorbent structure (18,68), comprising: a batt (21,72) of absorbent material, the batt being shaped to form:
a front region (30) terminating in a first longitudinal end (31);
a back region (32) terminating in a second longitudinal end (33);
a crotch region (35) located between the front and back regions (30,32); and
first and second sides (37,39) extending between the first and second longitudinal ends (31,32); and
wherein regions of relatively high compression (50) of the batt extend substantially continuously along each of the first and second sides (37,39), the regions (50) of relatively high compression being located inboard of each of the first and second sides (37,39) and tracking the contours of the respective first and second sides (37,39), the regions (50) of relatively high compression having a greater density and a reduced thickness compared to portions of the batt located inboard of the regions (50) of relatively high compression.

2. The shaped article of claim 1, wherein:
the first and second longitudinal ends (31,33) and the first and second sides (37,39) define a rim (16) extending completely around the batt (21,72); and
a region (50) of relatively high compression of the batt extends substantially continuously around the rim (16), the region (50) of relatively high compression being located inboard of the rim (16) and tracking the shapes of the first and second longitudinal ends (31, 33) and the contours of the first and second sides (37,39), the region (50) of relatively high compression having a greater density and a reduced thickness compared to portions of the batt located inboard of the region of relatively high compression.

3. The shaped article of claim 1 or 2 wherein each of the first and second sides (37,39) has a curved shape.

4. The shaped absorbent structure of any one of the preceding claims, wherein the absorbent material comprises an airlaid mixture of wood pulp fluff and high-absorbency material.

5. The shaped absorbent structure of any one of the preceding claims, wherein the regions (50) of relatively high compression are located from 0 to 50 millimeters from the respective first and second sides (37,39).

6. The shaped absorbent structure of claim 5, wherein the regions (50) of relatively high compression are located from 0.5 to 10 millimeters from the respective first and second sides (37,39).

7. The shaped absorbent structure of any one of the preceding claims, wherein:
the regions (50) of relatively high compression have a density of from 0.05 to 2.0 grams per cubic centimeter; and
the portions of the batt (21,72) located inboard of the regions of relatively high compression have a density of from 0.01 to 1.0 grams per cubic centimeter.

8. The shaped absorbent structure of any one of the preceding claims, wherein:
the regions (50) of relatively high compression have a thickness of from 0.1 to 10 millimeters; and
the portions of the batt (21,72) located inboard of the regions (50) of relatively high compression have a thickness of from 1.0 to 100 millimeters.

9. The shaped absorbent structure of any one of the preceding claims, wherein the regions (50) of relatively high compression extend continuously along each of the first and second sides (37,39).

10. The shaped absorbent structure of any one of the preceding claims, wherein the batt has a length of from 10 to 30 centimeters, a width of from 2.5 to 20 centimeters, and a thickness of from 3 to 40 millimeters.

11. The shaped absorbent structure of claim 2 or any claim dependent on claim 2, wherein the region (50) of relatively high compression extends continuously around the rim (16).

12. The shaped absorbent structure of any one of the preceding claims, wherein the first and second longitudinal ends (31, 33) have a curved shape.

13. The shaped absorbent structure of any one of the preceding claims, further comprising:
a pledget (74) of absorbent material, the pledget being generally smaller than the batt (72).

14. The shaped absorbent structure of claim 13 further comprising:
a tissue layer (71) bonded to the batt (72) at the region (50) of relatively high compression, the pledget (74) being sandwiched between the batt (72) and the tissue layer (71).

15. The shaped absorbent structure of claims 13 and 14, wherein the pledget (74) comprises wood pulp fluff and high-absorbency material (76).

16. The shaped absorbent structure of any one of claims 13 to 15, wherein the pledget (74) is wrapped in a tissue sheet (78).

17. The shaped absorbent structure of claim 13 or any claim dependent on claim 13, wherein:
the batt (72) is shaped to form a pocket or channel (82) extending between the front and back regions (30,32); and
the pledget (74) is positioned in the pocket or channel (82).

18. The shaped absorbent product of claim 2 or any claim dependent on claim 2 wherein:
the region (50) of relatively high compression is located from 0.5 to 10 mm from the rim.

19. An absorbent product (10,66,80), comprising:
a liquid-impervious, flexible outer shell (12);
a liquid-pervious body side liner (22) attached to the shell; and
a shaped absorbent structure (18,68) according to any one of the preceding claims being sandwiched between the shell (12) and the liner (22).

20. The absorbent product of claim 19, wherein the outer shell (12) is formed from a flexible polymeric foam material.

21. The absorbent product of claim 19 or 20, wherein the region of relatively high compression (50) is spaced apart from the liner (22).

22. The absorbent product of any one of claims 19 to 21, further comprising an acquisition layer (20) located between the liner (22) and the absorbent structure (18,68), the acquisition layer (20) being capable of being desorbed by the absorbent structure (16,68).

23. The absorbent product of claim 22, wherein the region of relatively high compression (50) is spaced apart from the liner(22) and the acquisition layer (20).

24. The absorbent product of any one of claims 19 to 23, wherein:
the shell (12) is formed of a polymeric foam material and has a length of from 10 to 30 centimeters, a width of from 3 to 18 centimeters, and a depth of from 0.6 to 6 centimeters, the shell defining a basin having a length, width, and a volume.

25. The absorbent product of any one of claims 19 to 24, wherein:
the body side liner (22) has a passage volume of 40 percent.

26. The absorbent product of any one of claims 19 to 25, wherein:
the shaped absorbent structure (18, 68) fills from 10 to 95 volume percent of the volume of the basin defined by the shell (12).

27. The absorbent product of any one of the preceding claims, wherein:
the acquisition layer (20) comprises a fibrous web of dry resilient fibers, at least about 30 weight percent of the fibers being hydrophilic, the acquisition layer filling from 5 to 30 volume percent of the volume of the basin defined by the shell (12).

28. A method of forming a shaped absorbent structure, comprising the steps of:
providing a web of absorbent material;
selecting a desired shaped structure to be separated from the web, the desired shaped structure having a front region terminating in a first longitudinal end, a back region terminating in a second longitudinal end, a crotch region between the front and back regions, and first and second sides extending between the longitudinal ends, the first and second longitudinal ends having preferably curved shapes, and each of the first and second sides having preferably a curved shape, the first and second longitudinal ends and the first and second sides defining a rim extending completely around and defining the desired shaped structure;
forming a region of relatively high compression in the web, the region of relatively high compression:
extending substantially continuously around the rim;
being located inboard of the rim;
tracking the shapes of the first and second longitudinal ends and the first and second sides; and
having a greater density and a reduced thickness compared to portions of the web located inboard of the region of relatively high compression; and
cutting the web along the rim to separate the desired shaped structure from the web.
